(19) **Europäisches Patentamt / European Patent Office / Office européen des brevets**

(11) **EP 2 087 909 B1**

(12) **FASCICULE DE BREVET EUROPEEN**

(45) Date de publication et mention
de la délivrance du brevet:
**13.03.2013 Bulletin 2013/11**

(51) Int Cl.:
*A61K 9/00* (2006.01)     *A61K 31/167* (2006.01)
*A61K 47/12* (2006.01)

(21) Numéro de dépôt: **09152160.9**

(22) Date de dépôt: **05.02.2009**

(54) **Formulation d'une solution de paracétamol injectable, procédé de préparation et de conditionnement d'une telle solution et dispositif de conditionnement d'une telle solution**

Formel für eine injizierbare Paracetamollösung, Herstellungs- und Verpackungsverfahren dieser Lösung und Verpackungsvorrichtung für diese Lösung

Formulation of an injectable paracetamol solution, method of manufacturing and packaging such a solution and device for packaging such a solution

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **06.02.2008 FR 0850742**

(43) Date de publication de la demande:
**12.08.2009 Bulletin 2009/33**

(73) Titulaire: **Moly Pharma**
**31470 Cambernard (FR)**

(72) Inventeur: **Douleau, Didier**
**31470 CAMBERNARD (FR)**

(74) Mandataire: **Schmit, Christian Norbert Marie**
**SCHMIT CHRETIEN**
**8, place du Ponceau**
**95000 Cergy (FR)**

(56) Documents cités:
**EP-A- 1 752 139        EP-A- 1 889 607**
**WO-A-01/93830        WO-A-98/05314**
**WO-A-2004/071502**

## Description

La présente invention concerne une formulation simplifiée de paracétamol pour préparation injectable prête à l'usage. Selon l'invention, ladite solution de paracétamol est composée uniquement de paracétamol, d'eau bi-distillée et d'un agent tampon. L'invention concerne également un procédé de préparation et de conditionnement d'une telle solution et un dispositif de conditionnement d'une telle solution.

Le paracétamol, comme de nombreux principes actifs, est sensible à la présence d'oxygène de l'air ainsi qu'à l'oxygène dissous dans les solvants aqueux classiques utilisés pour la préparation de solution injectable. La stabilité de la solution dépend en outre des conditions de pH du milieu qui peuvent évoluer au cours du temps par réaction avec certains composés chimiques entrant dans la composition des articles de conditionnement de la solution.

La dégradation chimique du paracétamol passe par la formation de 4-paraminophénol, de 3-paraminophénol qui se transforment en dérivés quinoniques colorés. La dégradation du paracétamol peut aboutir à des formes dimérisées du paracétamol.

Pour surmonter ce problème de stabilité, plusieurs méthodes ont été utilisées qui consistent à :

- neutraliser l'oxygène dissous dans la solution par adjonction à cette dernière d'agents antioxydants tels que l'acide ascorbique ou cystéine;
- éliminer l'oxygène dissous par un barbotage dans la solution d'un gaz inerte tel que l'azote ou le dioxyde,
- stabiliser le pH de la solution par des mélanges tampons autour de 6.

De telles solutions sont par exemple divulguées dans WO98/05314.

Toutes ces méthodes antérieures ont une certaine efficacité, mais les solutions désoxygénées conditionnées contiennent toujours une quantité résiduelle d'oxygène dissous.

Les solutions de paracétamol de l'état de la technique peuvent présenter des effets irritants ou allergisants liés à la présence des agents antioxydants.

En outre, il est connu que certains antioxydants peuvent se dégrader sous l'effet de la chaleur. Aussi il est indispensable d'ajouter des composés tels que des sels de métaux divalents pour préserver l'intégrité des antioxydants.

Ainsi les formules existantes de paracétamol en solution injectable sont composées de nombreux adjuvants qui rendent la préparation de la solution complexe et coûteuse, en multipliant les occasions d'apport en oxygène lors de l'incorporation des matières dans la solution malgré les précautions prises à cet effet.

Par ailleurs, pour garantir la stabilité de la solution conditionnée, les dispositifs de conditionnement proposés sont généralement des flacons verre obturés au moyen d'un bouchon en élastomère.

Les solutions peuvent être également conditionnées en poches souples obturées par un bouchon en élastomère. Le conditionnement en poche souple permet d'assurer une sécurité de manipulation dans les centres de soins par rapport aux risques de casse des flacons verre, mais aussi la sécurité du patient par l'absence du risque d'entrée d'air dans la solution pendant l'administration du produit qui peut être à l'origine des infections nosocomiales et des risques d'embolie gazeuse.

Sur le plan de la technique de remplissage de ces deux types de conditionnements, le flacon verre oblige à préserver un espace d'air entre le liquide et le bouchon pour permettre l'opération de stérilisation et éviter l'explosion du flacon ou son débouchage accidentel pendant cette opération. Cet espace de tête doit être de l'ordre de 20 à 30ml et doit être rempli de gaz inerte ou mis sous vide.

La poche souple a pour avantage de permettre un remplissage plus important du volume utile et de réduire l'espace de tête à un volume de l'ordre de 5 à 10 ml sans nécessité de traiter le contenu gazeux de cet espace. En outre, la matière plastique permet de stériliser les poches sans risque d'explosion ou de fissuration.

Toutefois tous les dispositifs de conditionnement antérieurs proposés ont pour but principal de protéger la solution de l'environnement extérieur, ils ne permettent pas de résoudre le problème d'oxydation dû à l'oxygène dissous dans la solution.

L'objectif principal de la présente invention est de protéger la solution de paracétamol contre l'oxydation ultérieure par l'oxygène dissous dans la solution provenant des opérations de préparation de la solution.

Pour pallier les problèmes techniques décrits ci-dessus, le concept de la présente invention est basé sur :

- une formulation simplifiée de solution de paracétamol où le nombre de composants de matières premières est réduit pour permettre d'optimiser les opérations et le temps de préparation ;
- un procédé de préparation automatique intégrant le contrôle continu des paramètres de qualité de la solution tels que la teneur en oxygène, le titre paracétamol, et le pH ;
- un dispositif de conditionnement incorporant un absorbant d'oxygène qui permet de capter l'oxygène dissous dans la solution de paracétamol.

A cet effet, l'invention concerne une formulation d'une solution de paracétamol injectable prête à l'usage.

Selon l'invention, la formulation de la solution de paracétamol est composée uniquement de paracétamol, d'eau bi-distillée et d'un agent tampon, ladite formulation ayant une concentration maximale de paracétamol de 1g/100 ml, un pH compris entre 5,5 et 6,5, et une teneur en oxygène inférieure à 0,2 ppm.

Une telle formulation simplifiée permet d'optimiser les opérations et le temps de préparation de la solution. Elle permet une économie des coûts de fabrication par rapport aux formulations existantes. En outre le nombre réduit de matières premières dans la formulation, permet d'intégrer l'ensemble des opérations de préparation dans un réacteur étanche unique et par là même supprimer les risques d'apport d'oxygène et d'impuretés lors des opérations de préparation.

De préférence l'agent tampon choisi est un agent tampon acétate qui n'apporte dans la formulation proposée dans la présente invention que des composants chimiques identiques au radical Acétyle de la molécule de paracétamol.

Le mélange tampon acétate permet de stabiliser le pH de la solution entre 5,5 et 6,5 et de préférence à pH 6 pour optimiser la stabilité du paracétamol et d'autoriser un conditionnement de la solution en poche souple PVC qui peut au cours du temps entraîner une diminution du pH de la solution et influencer la stabilité du principe actif.

De préférence, ce mélange tampon acétate est composé d'acétate de sodium trihydrate et d'acide acétique glacial.

L'invention concerne également un procédé de préparation et de conditionnement d'une solution de paracétamol telle que décrite ci-dessus au moyen d'un réacteur étanche, ledit réacteur étant composé d'une cuve reliée de manière étanche à un circuit de distribution d'eau bi-distillée, un circuit d'injection de vapeur blanche d'eau bi-distillée, un circuit de dissolution de paracétamol et d'acétate de sodium, un microdoseur d'acide acétique glacial et un circuit de transfert de solution.

Selon l'invention, le procédé comporte les étapes suivantes consistant à :

a) introduire un volume d'eau bi-distillée dans la cuve via le circuit de distribution d'eau bi-distillée, ladite cuve étant préalablement nettoyée à la vapeur blanche d'eau bi-distillée ;

b) refroidir le volume d'eau jusqu'à une température comprise entre 30°C et 35°C ;

c) contrôler la teneur en oxygène dissous dans le volume d'eau et procéder à une désoxygénation au moyen de la vapeur blanche d'eau bi-distillée si la teneur en oxygène est supérieure à 0, 2 ppm ;

d) introduire par aspiration le paracétamol et l'acétate de sodium sous forme de poudre dans ledit circuit de dissolution, le paracétamol et l'acétate de sodium étant dissous ensuite dans le circuit de dissolution au moyen d'un ultra-disperseur ;

e) renvoyer le mélange obtenu à l'étape d) dans la cuve par l'intermédiaire du circuit de transfert,

f) ajuster le pH de la solution au moyen du microdoseur d'acide acétique glacial puis ajuster le volume final du mélange à l'aide d'eau bi-distillée ;

g) contrôler la teneur en oxygène de la solution et procéder à une désoxygénation au moyen de la vapeur blanche si la teneur en oxygène est supérieure à 0,2 ppm ;

h) faire passer la solution de paracétamol à travers un premier filtre de 0,45 $\mu$m puis un deuxième filtre de 0,22$\mu$m ;

i) procéder à l'étape de conditionnement consistant à remplir dans une poche souple la solution de paracétamol filtrée à l'étape h), et à enfermer de manière étanche ladite poche dans un suremballage avec un absorbant d'oxygène ; et

les étapes a) à h) étant réalisées dans ledit réacteur étanche sous atmosphère désoxygénée à la vapeur d'eau bi-distillée blanche.

Ainsi cette approche de simplifier la formulation de la solution de paracétamol décrit ci-dessus a permis de développer un procédé industriel simplifié dans lequel les différentes opérations de préparation sont combinées dans un seul équipement de préparation appelé ici par le terme réacteur étanche et également d'intégrer un contrôle continu des paramètres de qualité de la solution tels que la teneur en oxygène et le pH durant les étapes de préparation. Ce contrôle continu permet donc de corriger les paramètres en cas de besoin de manière à ce que la solution finale vérifie les conditions optimales de stabilité.

Un autre avantage technique porte sur le mode opératoire du procédé qui fait intervenir l'eau bi-distillée comme composant de formulation et la vapeur blanche d'eau bi-distillée comme :

- agent d'inertage de la solution en cours de préparation si c'est nécessaire ;
- gaz de transfert lors des opérations de remplissage de la cuve et de vidange en fin de préparation ;
- agent de nettoyage de l'équipement de préparation entre deux séries de production.

De préférence, les matières premières solides qui sont le paracétamol et l'acétate de sodium, introduites dans le circuit de dissolution auront été stockées avant leur utilisation dans un sachet étanche et désoxygéné en présence d'un capteur d'oxygène.

L'invention concerne également un dispositif de conditionnement d'une solution de paracétamol injectable prête à l'usage. Selon l'invention, il comprend

- un conditionnement primaire composé d'une poche souple composée d'au moins un compartiment, l'un des compartiments contenant ladite solution,
- un conditionnement secondaire servant de suremballage de ladite poche comprenant deux parois de matériaux dont les bords sont scellés pour assurer l'étanchéité du suremballage,
- un absorbant d'oxygène étant disposé dans ledit suremballage, ledit absorbant étant destiné à capter l'oxygène dissous de la solution et l'oxygène présent dans le suremballage.

Un des principaux avantages du dispositif de conditionnement qui vient d'être défini, réside dans la combinaison de trois composants de conditionnement : la poche, le suremballage et l'absorbant qui va amorcer une réaction de diffusion de l'oxygène dissous dans la solution de paracétamol conditionnée dans la poche à travers les parois de la poche et l'absorption de l'oxygène par des capteurs chimiques.

D'autres caractéristiques et avantages de l'invention seront apparents à la lecture de la description qui suit faite en référence aux dessins schématiques annexés donnés uniquement à titre d'exemple et dans lesquels:

- la figure 1 représente schématiquement les différentes étapes du procédé de préparation et de conditionnement d'une solution de paracétamol selon l'invention ;
- la figure 2 représente schématiquement le dispositif de conditionnement d'une solution de paracétamol injectable prête à l'usage selon l'invention ;
- la figure 3 représente schématiquement le processus d'absorption d'oxygène par l'absorbant d'oxygène.

Le tableau suivant présente les différents composants de la formulation simplifiée de la solution de paracétamol de l'invention :

| Composition | Fonction |
| --- | --- |
| Paracétamol | Principe actif |
| Acétate de sodium trihydrate | Agent tampon |
| Acide acétique glacial | Agent tampon |
| Eau bi-distillée | Solvant de dissolution et de dilution |

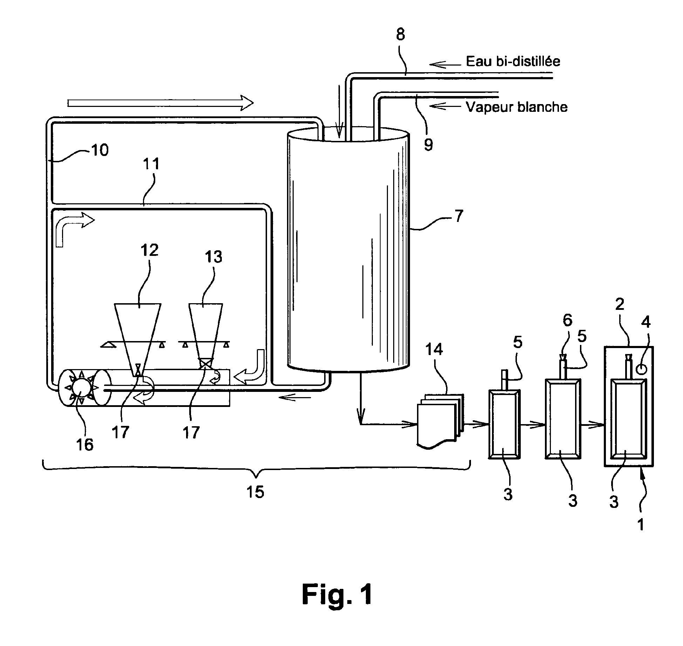

La figure 1 illustre le procédé de préparation d'une telle formulation simplifiée de la solution de paracétamol dans lequel les opérations de pesées des composants, de dispersion et de dissolution et de filtration sont intégrées dans un seul équipement étanche à l'environnement extérieur.

Cet équipement, appelé plus généralement réacteur 15 est composé d'une cuve 7 reliée de manière étanche à un circuit de distribution d'eau bi-distillée 8, un circuit d'injection de vapeur blanche d'eau bi-distillée 9, un circuit de dissolution de paracétamol et d'acétate de sodium 11 et un circuit de transfert de solution 10.

Avantageusement cet équipement est pourvu de moyens au niveau de la cuve 7 non illustrés sur la figure 1 qui permettent de contrôler en continu les différents paramètres de la solution tels que la teneur en oxygène, le titre paracétamol, et le pH. Les valeurs mesurées peuvent être envoyées de manière périodique vers une interface graphique ou transmises uniquement sous l'interrogation d'un opérateur.

Le procédé de la présente invention peut être appliqué pour des réacteurs de capacité allant de 100 litres à 5000 litres.

Le procédé comporte les étapes suivantes :

ETAPE 1 :

On remplit la cuve 7 d'eau bi-distillée servant de solvant de dissolution et de dilution via le circuit de distribution d'eau bi-distillée 8. Cette cuve est préalablement remplie de vapeur blanche injectée via le circuit 9 afin de supprimer toute trace d'air. De préférence, la cuve est remplie d'eau bi-distillée pour un volume équivalent à 90% du volume du mélange final.

ETAPE 2 :

L'eau bi-distillée est ensuite refroidie jusqu'à une température adaptée comprise entre 30°C et 35°C, de préférence à 30°C. On réalise ensuite un contrôle de la teneur en oxygène dissous dans le volume d'eau et une désoxygénation au moyen de la vapeur d'eau blanche si la teneur mesurée est supérieure à 0,2 ppm.

ETAPE 3 :

On procède à l'étape de dissolution des matières premières solides selon les opérations suivantes :

- on alimente en eau bi-distillée le circuit de dissolution 11 depuis la cuve 7 en mettant en route la pompe haut débit 16,
- le paracétamol et l'acétate sodium stockés sous forme de poudre dans les réservoirs 12, 13 sont pesés automatiquement,
- on aspire les matières pesées au travers d'une tuyère 17 reliée à l'ultra-disperseur 16 qui entraîne une dispersion à grande vitesse des poudres dans l'eau puis dissolution des produit dans le circuit 11.

ETAPE 4 :

Après dissolution des matières premières dans le circuit 11, ce dernier est raccordé au circuit de transfert 10 pour transférer la solution de paracétamol dans la cuve de mélange final 7. Après contrôle du pH, ce dernier est ajusté à 6 par un apport automatique d'acide acétique et le volume final du mélange sera ajusté par un apport d'eau bi-distillée.
La teneur en oxygène est contrôlée de nouveau et réajustée pour se situer à une teneur au moins inférieure à 0,2 ppm. Le mélange final est maintenu sous agitation jusqu'à la réalisation des contrôles de conformité.

ETAPE 5 :

Avant de conditionner la solution, elle est soumise à une filtration 14 de 0,45 $\mu$m suivie d'une deuxième filtration de 0,22 $\mu$m qui est une filtration stérilisante.
L'ensemble des opérations qui sont décrites ci-dessus est effectué sous atmosphère désoxygénée à la vapeur blanche d'eau bi-distillée.

ETAPE 6 :

La dernière étape du procédé concerne le conditionnement de la solution finale qui comprend les opérations suivantes :

- on remplit dans une poche souple 3 la solution de paracétamol filtrée, dans le cas où la poche souple comporte deux compartiments, en parallèle du remplissage de l'un des compartiments de la solution, on procède également au remplissage du deuxième compartiment d'un autre composé tel que la codéine ou le Kétoprofène, immédiatement après le remplissage, l'ouverture tubulaire 5 de la poche est bouchée par une obturateur 6 telle qu'une capsule quart de tour;
- on enferme ensuite la poche souple 3 dans un suremballage 2 avec un absorbant d'oxygène 4 et le suremballage est scellé pour assurer l'étanchéité de l'ensemble.

De préférence, l'étape de conditionnement de la solution est réalisée dans une salle de classe particulaire A ou B (3500 particules au m3) de manière à éviter l'étape de stérilisation par autoclavage.
Dans le cas où l'étape de conditionnement est réalisée dans une salle de classe particulaire C, le procédé comprend une étape supplémentaire de stérilisation par autoclavage de la solution conditionnée, à savoir elle est soumise à un chauffage à 121 °C pendant 15 mn.

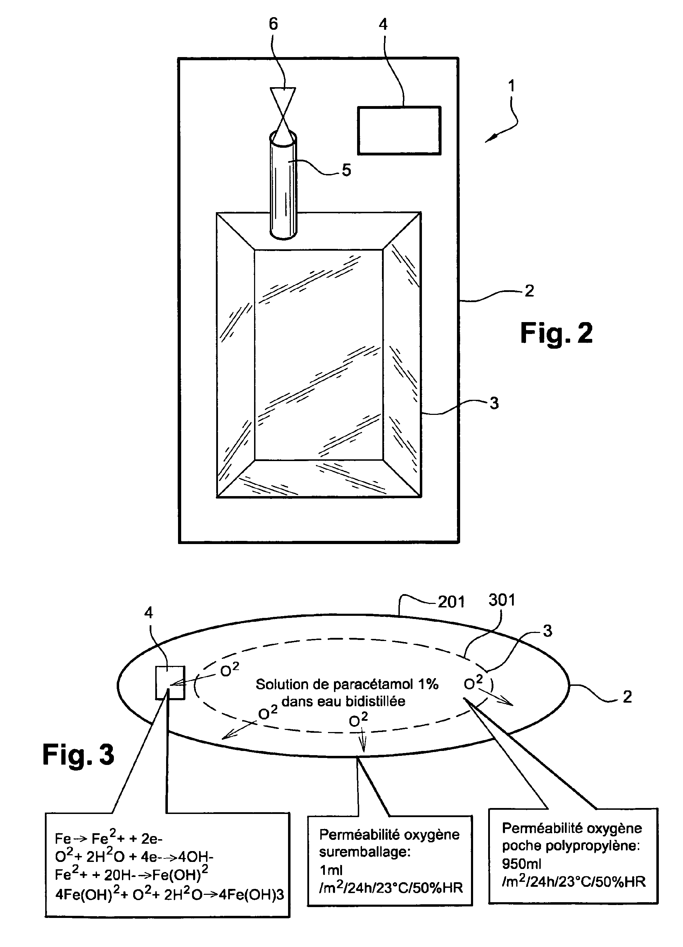

La figure 2 illustre un mode préféré de réalisation du dispositif de conditionnement d'une solution de paracétamol. Il comprend une poche souple 3 dans laquelle est conditionnée la solution de paracétamol. Afin de protéger la solution contre l'air extérieur, la poche est disposée dans un suremballage 2 ainsi qu'un absorbant d'oxygène 4.
Généralement, cet absorbant d'oxygène se présente sous la forme d'un sachet ou de capsule contenant un mélange de matière minérale à base d'oxydes métalliques, de préférence à base d'oxyde de fer ne présentant aucune toxicité à l'usage alimentaire.
Pour assurer l'efficacité du dispositif de conditionnement et augmenter la durée de conservation de la solution, on réalise un vide partiel dans l'espace entre la poche et le suremballage avant de sceller le suremballage.
Le suremballage est réalisé de préférence dans un matériau complexe polyester/polyamide/Aluminium/polypropylène qui est donc opaque. Toutefois le suremballage peut être également réalisé dans un matériau complexe polyester/polypropylène, et les résultats de l'étude III décrits ci-dessous montrent que la stabilité de la solution n'est pas affectée par le fait d'utiliser un tel matériau. De préférence, les parois du suremballage présentent une perméabilité à l'oxygène au moins inférieure à 1cm$^3$/m$^2$/24h à T = 23°C et dans une humidité ambiante relative de 50%HR.

La poche souple est en matière plastique choisie dans un groupe comprenant du polychlorure de vinyle, du polypropylène ou un matériau complexe multicouche à base de polypropylène. De préférence, les parois de la poche présentent une perméabilité à l'oxygène comprise entre 950cm$^3$/m$^2$/24h et 1000 cm$^3$/m$^2$/24h à T = 23°C et dans une humidité ambiante relative de 50%HR.

La matière antioxydant contenue dans le sachet en présence d'un faible taux d'humidité va engager une réaction de fixation chimique de l'oxygène gazeux présent dans l'environnement. La capacité d'absorption est modulable et se situe entre 100 et 400 ml d'oxygène.

La figure 3 illustre le principe de la réaction chimique qui est le suivant :

$$Fe \rightarrow Fe^{2+} + 2\ e^-$$

$$O^2 + 2H^2O + 4\ e^- \rightarrow 4OH^-$$

$$Fe^{2+} + 2OH^- \rightarrow Fe\ (OH)^2$$

$$4Fe(OH)^2 + O^2 + 2H^2O \rightarrow 4\ Fe(OH)^3$$

L'innovation se **caractérise en ce que** le capteur 4 est localisé entre la paroi de la poche contenant la solution de paracétamol et la paroi du suremballage tel que présenté dans la figure 2. Le différentiel de perméabilité aux gaz, notamment à l'oxygène entre les parois 301 de la poche souple 3 et les parois 201 du suremballage 2 va entraîner le passage de l'oxygène dissous de la solution vers le milieu contenant l'absorbant 4.

La suite de la description porte sur les résultats de trois études réalisées sur des prototypes de solutions préparées selon le procédé tel que décrit ci-dessus.

**Etude I : Vérification de la faisabilité de la formule et de son conditionnement avec ou sans capteur d'oxygène**

Le tableau suivant résume les principaux paramètres de mesures lors des différentes opérations de préparation de la solution :

| Opération | Tests | Résultats |
|---|---|---|
| Alimentation du réacteur avec eau bi-distillée | Volume<br>Température<br>Refroidissement<br>Taux oxygène | 89 litres<br>75°c<br>31 °c<br>0,07mg/l |
| Incorporation du Paracétamol | Temps de dissolution<br>Limpidité | 10 minutes<br>Solution limpide |
| Ajustement du volume final avec eau bi-distillée | Volume | 11 litres |
| Ajustement du pH | Mesure du pH<br>Quantité Acétate de sodium | 6,0 |
| Ajustement du pH final avec acide acétique | Taux oxygène Densité | 0,04mg/l 1,0069 |
| Filtration 0,22μm | Contrôle de particules | 0 |
| Remplissage Poches Polypropylène | Contrôle volume | 100ml |

Dans cet essai, le mélange final de 100 litres a été conditionné en poches polypropylène de 100 ml sur des équipements industriels semi-automatiques implantés en zone pharmaceutique de classe C sans protection de flux laminaire de gaz inerte.

Ces poches une fois remplies et bouchées ont été divisées en deux lots de conditionnement secondaire :

- Lot 10788-01 (suremballage de la poche sans incorporation d'un absorbant d'oxygène),
- Lot 10788-02 (suremballage de la poche avec absorbant d'oxygène ayant une capacité d'absorption de 400ml)
Le matériau utilisé pour réaliser le suremballage est identique pour les deux lots, à savoir un matériau complexe polyester/polyamide/aluminium/polypropylène.

Le matériau de l'absorbant d'oxygène est un mélange d'oxyde minéraux.
Ces deux lots ont été analysés, stérilisés à 121°c pendant 15 minutes puis mis en stabilité après un nouveau contrôle analytique selon le protocole suivant :

a) Température de stockage : 55°c. Durée 30 jours. Périodicité : T0, T+15, T+30
b) Température de stockage : 40°c. Durée 90 jours. Périodicité T0, T+30, T+60, T+90
c) Température de stockage : 25°c Durée 9 mois. Périodicité T0, T+6 mois, T+9 mois

Les caractéristiques observées pour démontrer la qualité de la formule du procédé et du conditionnement ont été : le titre en paracétamol, l'aspect coloré de la solution, le pH, et le dosage des impuretés totales.
Nous avons choisi l'indice de coloration comme facteur discriminant de ces essais car la déstabilisation du Paracétamol en présence d'oxygène passe par l'apparition du paraminophénol instable qui se transforme en dérivés quinoniques colorés.
Le second critère discriminant concerne le titre en impuretés totales par rapport au Paracétamol mais les premiers mois de notre test n'ont pas permis de contrôler ces impuretés faute de méthode analytique pertinente et validée. Ce n'est qu'à partir du troisième mois que ce deuxième critère a été intégré dans notre analyse.
Le tableau ci-après, illustre les conclusions exposées ci-dessous :

| Détermination | Coloration (absorbance à 430 nm) | Impuretés totales en % de paracétamol | pH 5,5 à 6,5 | Titre en Paracétamol 0,950g/100ml à 1,050g/100ml |
|---|---|---|---|---|
| Poche avant autoclave | 0 | / | 6,0 | 0,986 |
| T0 sans capteur après autoclave | 0,009 | / | 6,0 | 1,023 |
| T0 avec capteur après autoclave | 0,009 | / | 6,0 | 1,023 |
| 55°c T+15 sans capteur | 0,019 | / | 6,0 | 1,003 |
| 55°c T+15 avec capteur | 0,007 | / | 6,0 | 1,014 |
| 55°c T+30 sans capteur | 0,029 | / | 6,0 | 0,988 |
| 55°c T+30 avec capteur | 0,006 | / | 6,0 | 0,999 |
| 40°c T+30 sans capteur | 0,013 | / | 6,0 | 0,985 |
| 40°c T+30 avec capteur | 0,007 | / | 6,0 | 0,991 |
| 40°c T+60 sans capteur | 0,020 | / | 6,0 | 1,022 |
| 40°c T+60 avec capteur | 0,007 | / | 6,0 | 1,034 |
| 40°c T+90 sans capteur | 0,025 | 0,095 | 6,0 | 1,007 |
| 40°c T+90 avec capteur | 0,007 | 0,094 | 6,0 | 1,006 |

(suite)

| Détermination | Coloration (absorbance à 430 nm) | Impuretés totales en % de paracétamol | pH 5,5 à 6,5 | Titre en Paracétamol 0,950g/100ml à 1,050g/100ml |
|---|---|---|---|---|
| 25°c T+ 6mois sans capteur | 0,014 | 0,134 | 6,0 | 1,030 |
| 25°c T+ 6mois avec capteur | 0,010 | 0,128 | 6,0 | 1,040 |
| 25°c T+ 9mois sans capteur | 0,019 | 0,152 | 6,0 | 1,020 |
| 25°c T+ 9mois avec capteur | 0,009 | 0,134 | 6,0 | 1,040 |

A l'examen des résultats de l'étude de stabilité, il apparaît clairement que :

- la formule simplifiée de paracétamol est stable dans les conditions de températures les plus difficiles, les variations de la teneur en paracétamol sont comprises dans la variabilité de la méthode analytique ;
- une différence significative de l'indice de coloration en faveur des solutions conditionnées dans des suremballages munis de capteurs d'oxygène. Ce phénomène s'explique par la différence de teneur en impureté 4-aminophénol trouvée dans les deux catégories de poches. Les poches avec capteur contiennent du paraminophénol stabilisé par absence d'oxygène, alors que les poches sans capteur n'ont plus de paraminophénol qui a été transformé par la présence d'oxygène non capturé ;
- on constate qu'après un séjour de 90 jours à la température de 40°c le pourcentage des impuretés totales est identique entre les poches munies de capteur d'oxygène et les poches sans capteur ;
- on constate qu'après une mise en stabilité de 9 mois à 25°c, il apparaît une différence significative des taux d'impuretés entre les deux lots de poches et en faveur des poches avec capteurs d'oxygène ;
- le pH reste stable quelles que soient les conditions étudiées.

Les résultats de cet essai montrent de manière inattendue que la stabilité de la solution de paracétamol prête à l'usage selon la présente invention peut être obtenue en absence d'excipient, contrairement aux solutions de l'état de la technique. La formule simplifiée, un procédé de préparation intégrant l'ensemble des opérations de préparation dans un équipement étanche à l'environnement extérieur ainsi qu'un dispositif de conditionnement muni d'un absorbant permettent de stabiliser le paracétamol.

**Etude II : Stabilité de la solution en fonction du pH**

Le second essai a pour but de démontrer que le pH est un paramètre critique de conception de la formule, et que le pH 6 présente toutes les garanties pour une bonne stabilité de la formule.
Le protocole de l'essai consiste à injecter dans des poches du soluté PROTOTYPE-1 préalablement stockées à température ambiante, des doses d'acide chlorhydrique ou de soude pour obtenir des poches dont le pH se situe entre 4,5 et 7,5. Après injection des acidifiants ou des alcalinisants, une obturation de l'ouverture faite par l'aiguille dans les bouchons a été effectuée avec les matériaux utilisés pour la fabrication des bouchons de poche perfusion.
Ces échantillons ont été placés en étuve à 55°c pour une durée de 30 jours et ont été analysés à 15 jours et à 30 jours.

| Détermination | | Coloration (absorbance à 430 nm) | Impuretés totales en % de paracétamol | pH | Titre en paracétamol (g/100ml) |
|---|---|---|---|---|---|
| pH 4,5 | T+15 jours | 0,071 | 0,266 | 4,51 | 1,02 |
| pH 4,5 | T+ 30jours | 0,139 | 0,284 | 4,55 | 1,05 |
| pH 5,5 | T+15 jours | 0,043 | **0,243** | 5,44 | 1,02 |
| pH 5,5 | T+30 jours | 0,067 | **0,319** | 5,44 | 1,05 |
| pH 6 | T+15 jours | **0,035** | 0,331 | 6,03 | 1,01 |

(suite)

| Détermination | | Coloration (absorbance à 430 nm) | Impuretés totales en % de paracétamol | pH | Titre en paracétamol (g/100ml) |
|---|---|---|---|---|---|
| pH 6 | T+30 jours | **0,052** | 0,435 | 6,02 | 1,05 |
| pH 6,5 | T+15 jours | 0,046 | 0,483 | 6,51 | 1,04 |
| pH 6,5 | T+30 jours | 0,059 | 0 ,667 | 6,47 | 1,04 |
| pH 7,5 | T+15 jours | 0,107 | 0,898 | 7,21 | 1,05 |
| pH 7,5 | T+30 jours | 0,121 | 1,534 | 7,00 | 1,02 |

On peut noter qu'au-delà de pH 6,5, on observe une forte dégradation manifestée par une coloration très prononcée de la solution ainsi qu'un taux d'impuretés important.

Dans les zones d'acidité, à savoir de pH 4,5 ; on observe une très forte coloration mais un niveau d'impuretés très bas.

Conclusion :

Notre choix du tampon pH 6 est le meilleur compromis de la série avec un indice de coloration le plus bas et une évolution du taux d'impuretés le plus lent.

**Etude III : Influence des matériaux du suremballage sur la stabilité de la solution**

L'objectif de cette troisième étude a pour but de vérifier la faisabilité technique de l'utilisation des matériaux Polyester/Polypropylène pour réaliser le suremballage. En effet, en plus de leur imperméabilité aux gaz et notamment à l'oxygène, ils sont transparents, permettant ainsi d'assurer un contrôle visuel après suremballage au niveau de la production mais également au niveau de l'utilisation par les infirmières.

Nous avons pour ce test réduit la capacité d'absorption de 400 ml d'oxygène à 100 ml pour vérifier la perméabilité de la poche perfusion et le comportement de la solution de paracétamol. La taille du lot est de 100 litres et il a été conditionné sans condition particulière de protection sous gaz inerte en trois lots :

- Lot 1 suremballé en complexe aluminium avec capteur d'oxygène 100ml
- Lot 2 suremballé sous complexe aluminium sans capteur,
- Lot 3 suremballé sous complexe polyester/Polypropylène avec capteur d'oxygène 100ml

Ces trois lots, après stérilisation à 121 °c 15 minutes ont été mis en étuve à 55°c pendant un mois et analysés avec les mêmes protocoles que ceux utilisés dans la première étude et les paramètres discriminants ont été la coloration de la solution et le taux d'impuretés totales.

Le tableau ci-après illustre les conclusions présentées ci-dessus :

| Détermination | Coloration (absorbance à 430 nm) | Impuretés totales en % de paracétamol | pH | Titre en paracétamol (g/100ml) |
|---|---|---|---|---|
| **Complexe Alu avec Capteur** | | | | |
| 55°c T-0 | 0,004 | 0,07% | 5,92 | 1,03 |
| 55°c T+7 | 0,004 | 0,06% | 5,90 | 1,01 |
| 55°c T+15 | 0,003 | 0,06% | 5,89 | 1,03 |
| 55°c T+30 | 0,004 | 0,06% | 5,90 | 1,03 |
| **Complexe Alu sans capteur** | | | | |
| 55°c T-0 | 0,004 | 0,07% | 5,92 | 1,03 |
| 55°c T+7 | 0,004 | 0,08% | 5,91 | 1,01 |
| 55°c T+15 | 0,015 | 0,09% | 5,90 | 1,02 |

(suite)

| Complexe Alu sans capteur | | | | |
|---|---|---|---|---|
| 55°c T+30 | 0,030 | 0,13% | 5,90 | 1,03 |
| **Complexe polypropylène avec capteur** | | | | |
| 55°c T-0 | 0,004 | 0,06% | 5,90 | 1,03 |
| 55°c T+7 | 0,006 | 0,07% | 5,92 | 1,02 |
| 55°c T+15 | 0,005 | 0,06% | 5,90 | 1,02 |
| 55°c T+30 | 0,008 | 0,07% | 5,90 | 1,05 |

L'examen de cet essai confirme bien de l'intérêt du capteur d'oxygène pour assurer l'absorption de l'oxygène dissous dans le paracétamol et montre qu'il est possible de sécuriser la conservation de la solution par des capteurs dont la capacité d'absorption se situe dans une échelle de 100ml à 400ml d'oxygène.

Cet essai démontre qu'il est possible d'utiliser un matériau de suremballage transparent à la place de complexes Aluminium à la condition d'avoir un différentiel de perméabilité aux gaz entre la poche perfusion et la sache de suremballage de l'ordre de 900.

Les tableaux ci-après montrent les résultats d'une étude de stabilité accélérée entre une solution simplifiée de paracétamol obtenue selon l'invention et une solution commercialisée telle que Perfalgan.

Impureté K = 4 aminophénol, NP : non réalisé ND : non détecté RT : seuil de report (0.05%)

| STOCKAGE A +55°c | Temps initial | Paracétamol 1 g IV Poche pour perfusion | | | Solution commercialisée | | |
|---|---|---|---|---|---|---|---|
| Description de l'emballage | | poche flexible de 100 ml suremballée dans une poche en aluminium avec un absorbeur d'oxygène | | | Bouteille en verre de 100 ml bouchée avec un bouchon en élastomère | | |
| DETERMINATIONS | Spécifications pour la solution de l'invention | 801101 | 801401 | 801402 | 07G1301 | 8D34475 | 8F34140 |
| ESSAIS PHARMACEUTIQUES Caractères | Limpide et pas plus colorée que J5 et R6 | Conform | Conform | Conform | Conform | Conform | Conform |
| Coloration à 430 nm | Pour information | 0.005 | 0.004 | 0.007 | 0.003 | 0.003 | 0.001 |
| ESSAIS pH Densité Osmolalité (mOsm/Kg) | 5.5-6.5 <br> 270.0-310.0 | 6.0 <br> 1.002 <br> 288.0 | 6.0 <br> 1.007 <br> 293.0 | 6.0 <br> 1.005 <br> 288.7 | 5.6 <br> 1.014 <br> 291.7 | 5.6 <br> NP <br> NP | 5.7 <br> 1.015 <br> 292.0 |
| DOSAGE Paracétamol (mg/ml) | 9.50 - 10.50 | 10.00 | 10.29 | 10.23 | 10.08 | 9.79 | 9.84 |
| ESSAIS de PURETE (%) <br><br> Impuretés K Paracétamol dimère <br> Impureté individuelle inconnue <br> Impuretés totales inconnues | $\leq 0.05$ <br> $\leq 0.05$ <br> $\leq 0.10$ <br> $\leq 0.10$ | 0.01 <br> 0.01 <br> < RT <br> < RT | 0.01 <br> 0.01 <br> < RT <br> < RT | 0.01 <br> 0.01 <br> < RT <br> < RT | 0.01 <br> ND <br> < RT <br> < RT | 0.02 <br> ND <br> < RT <br> < RT | 0.02 <br> ND <br> < RT <br> < RT |

| STOCKAGE A +55°C | T + 14 JOURS | Paracétamol 1 g/100 ml Poche de perfusion | | | Solution commercialisée | | |
|---|---|---|---|---|---|---|---|
| Description de l'emballage | | poche flexible de 100 ml suremballée dans une poche en aluminium avec un absorbeur d'oxygène | | | Bouteille en verre de 100 ml bouchée avec un bouchon en élastomère | | |
| DETERMINATIONS | Spécifications pour la solution de l'invention | 801101 | 801401 | 801402 | 07G1301 | 8D34475 | 8F34140 |
| ESSAIS PHARMACEUTIQUES Caractères | Limpide et pas plus colorée que J5 et R6 | NP | Conform | Conform | Conform | Conform | Conform |
| Coloration à 430 nm | Pour information | NP | 0.004 | 0.007 | 0.001 | 0.002 | 0.001 |
| ESSAIS pH | 5.5 - 6.5 | NP | 6.0 | 6.0 | 5.6 | 5.4 | 5.7 |
| Densité | | NP | 1.007 | NP | 1.013 | NP | 1.014 |
| Osmolalité(mOsm/Kg) | 270.0-310.0 | NP | 295.0 | NP | 294.7 | NP | 288.7 |
| DOSAGES Paracétamol(mg/ml) | 9.50 - 10.50 | NP | 10.33 | 10.3 | 10.15 | 9.91 | 9.86 |
| ESSAIS DE PURETE (%) | ≤ 0.05 | NP | 0.03 | 0.02 | 0.03 | 0.04 | 0.03 |
| | ≤ 0.05 | NP | 0.01 | 0.01 | ND | ND | ND |
| Impureté K | ≤ 0.10 | NP | < RT | < RT | < RT | < RT | < RT |
| Paracétamol dimère Impureté individuelle inconnue | ≤ 0.10 | NP | < RT | < RT | < RT | < RT | < RT |
| Impuretés totales Inconnues | | | | | | | |

| STOCKAGE A +55°C | T + 21 jours | Paracétamol 1 g/100 ml Poche de perfusion | | | Solution commercialisée | | |
|---|---|---|---|---|---|---|---|
| Description de l'emballage | | poche flexible de 100 ml suremballée dans une poche en aluminium avec un absorbeur d'oxygène | | | Bouteille en verre de 100 ml bouchée avec un bouchon en élastomère | | |
| DETERMINATIONS | Spécifications pour la solution de l'invention | 801101 | 801401 | 801402 | 07G1301 | 8D34475 | 8F34140 |
| ESSAIS PHARMACEUTIQUES Caractères | Limpid et pas plus colorée que J5 et R6 | Conform | Conform | Conform | Conform | Conform | Conform |

(suite)

| STOCKAGE A +55°C | T + 21 jours | Paracétamol 1 g/100 ml Poche de perfusion | | | Solution commercialisée | | |
|---|---|---|---|---|---|---|---|
| Description de l'emballage | | poche flexible de 100 ml suremballée dans une poche en aluminium avec un absorbeur d'oxygène | | | Bouteille en verre de 100 ml bouchée avec un bouchon en élastomère | | |
| DETERMINATIONS | Spécifications pour la solution de l'invention | 801101 | 801401 | 801402 | 07G1301 | 8D34475 | 8F34140 |
| **ESSAIS PHARMACEUTIQUES** Coloration à 430 nm | Pour information | 0.004 | 0.007 | 0.015 | Non mesurable | 0.014 | 0.001 |
| **ESSAIS** pH Densité Osmolalité(mOsm/Kg) | 5.5 - 6.5 270.0-310.0 | 6.0 1.008 285.0 | 6.0 1.008 294.5 | 6.0 NP NP | 5.5 1.016 295.5 | 5.3 NP NP | 5.6 1.015 289.5 |
| **DOSAGE** Paracétamol (mg/ml) | 9.50 - 10.50 | 10.03 | 10.18 | 10.27 | 10.04 | 9.85 | 9.74 |
| ESSAIS DE PURETE (%) Impureté K Paracétamol dimère Impureté individuelle inconnue Impuretés totales Inconnues | $\leq$ 0.05 $\leq$ 0.05 $\leq$ 0.10 $\leq$ 0.10 | 0.03 0.02 < RT < RT | 0.03 0.01 < RT < RT | 0.02 0.01 < RT < RT | 0.03 ND < RT < RT | 0.04 ND < RT < RT | 0.03 ND < RT < RT |

| STOCKAGE A +55°C | T + 3 mois | Paracétamol 1 g/100 ml Poche de perfusion | | | Solution commercialisée | | |
|---|---|---|---|---|---|---|---|
| **Description de l'emballage** | | poche flexible de 100 ml emballée dans une poche en aluminium avec un absorbant d'oxygène | | | Bouteille en verre de 100 ml bouchée avec un bouchon en élastomère | | |
| DETERMINATIONS<br><br>**ESSAIS**<br><br>**PHARMACEUTIQUES**<br>Caractères<br><br>Coloration à 430 nm | Spécifications pour la solution de l'invention<br><br>Limpide et pas plus colorée que J5 et R6<br>Pour information | 801101<br><br><br><br>Conform<br><br>0.006 | 801401<br><br><br><br>Conform<br><br>0.005 | 801402<br><br><br><br>Conform<br><br>0.012 | 07G1301<br><br><br><br>Conform<br><br>Non mesurable | 8D34475<br><br><br><br>Conform<br><br>Non mesurable | 8F34140<br><br><br><br>Conform<br><br>0.002 |
| **ESSAIS**<br>pH<br>Densité<br>Osmolalité(mOsm/Kg) | 5.5 - 6.5<br><br>270.0-310.0 | 6.0<br>1.007<br>283.0 | 6.0<br>1.007<br>290.0 | 6.0<br>1.006<br>289.3 | 5.4<br>1.012<br>293.0 | 5.3<br>1.016<br>284.3 | 5.6<br>1.013<br>285.0 |
| **DOSAGE**<br>Paracétamol (mg/ml) | 9.50 - 10.50 | 9.86 | 10.10 | 10.17 | 9.89 | 9.79 | 9.58 |
| ESSAIS DE PURETE<br>**(%)**<br><br><br>Impureté K<br>Paracétamol dimère<br>Impureté individuelle inconnue<br>Impuretés totales Inconnues | $\leq 0.05$<br>$\leq 0.05$<br>$\leq 0.10$<br><br>$\leq 0.10$ | 0.06<br>< 0.01<br>< RT<br><br>< RT | 0.07<br>0.01<br>< RT<br><br>< RT | 0.08<br>0.01<br>< RT<br><br>< RT | 0.11<br>ND<br>< RT<br><br>< RT | 0.16<br>ND<br>< RT<br><br>< RT | 0.08<br>ND<br>< RT<br><br>< RT |

Cette étude de stabilité comparative entre une solution de paracétamol 1g et une solution Perfalgan montre deux principales différences :

- le taux d'impureté de 4 aminophénol augmente beaucoup plus vite dans une solution commercialisée actuellement telle que Perfalgan, et après 3 mois de stockage, le taux moyen obtenu dans cette solution classique est deux fois plus important que le taux moyen obtenu dans la solution de Paracétamol 1 g préparée selon l'invention,
- on détecte une faible présence de paracétamol dimère dans la solution de Paracétamol 1 g préparée selon l'invention, toutefois le taux n'augmente pas avec la durée de stockage,
- il ressort des résultats qu'il y a une légère variation de pH dans la solution de l'art antérieur alors que durant le même temps de stockage, la solution de paracétamol préparée selon l'invention présente une bonne stabilité de pH.

Pour les deux produits étudiés, les paramètres physico-chimiques tels que la densité et l'osmolalité ne montrent aucune variation et le contenu de principal actif est stable. Le taux des impuretés inconnus n'augmente pas durant le stockage et il reste très faible (environ 0.02 % pour les deux produits).

**Revendications**

1. Formulation d'une solution de paracétamol injectable prête à l'usage, **caractérisée en ce que** ladite solution de paracétamol est composée uniquement de paracétamol, d'eau bi-distillée et d'un agent tampon, ladite formulation ayant une concentration maximale de paracétamol de 1g/100 ml, un pH compris entre 5,5 et 6,5, et une teneur en oxygène inférieure à 0,2 ppm.

2. Formulation selon la revendication 1, **caractérisée en ce que** l'agent tampon est un couple agent tampon composé d'acétate de sodium et d'acide acétique.

3. Formulation selon la revendication 1 ou 2, **caractérisée en ce que** le pH de la solution est de 6.

4. Formulation selon l'une des revendications 1 à 3, **caractérisée en ce qu'**elle contient 1g/100ml de paracétamol.

5. Procédé de préparation et conditionnement d'une solution de paracétamol selon l'une des revendications 1 à 4 au moyen d'un réacteur étanche (15), ledit réacteur étant composé d'une cuve (7) reliée de manière étanche à un circuit de distribution d'eau bi-distillée (8), un circuit d'injection de vapeur blanche d'eau bi-distillée (9), un circuit de dissolution (11) de paracétamol et d'acétate de sodium et un circuit de transfert de solution (10), **caractérisé en ce que** ledit procédé comporte les étapes suivantes consistant à :

   a) introduire un volume d'eau bi-distillée dans la cuve (7) via le circuit de distribution d'eau bi-distillée (8), ladite cuve étant préalablement nettoyée à la vapeur blanche d'eau bi-distillée ;
   b) refroidir le volume d'eau jusqu'à une température comprise entre 30°C et 35 °C ;
   c) contrôler la teneur en oxygène dissous dans le volume d'eau et procéder à une désoxygénation au moyen de la vapeur blanche d'eau bi-distillée si la teneur en oxygène est supérieure à 0, 2 ppm ;
   d) introduire par aspiration le paracétamol et l'acétate de sodium sous forme de poudre dans ledit circuit de dissolution (11), le paracétamol et l'acétate de sodium étant dissous ensuite dans le circuit de dissolution au moyen d'un ultra-disperseur (16) ;
   e) renvoyer le mélange obtenu à la sortie du circuit de dissolution dans la cuve par l'intermédiaire du circuit de transfert (10),
   f) ajuster le pH de la solution au moyen d'acide acétique glacial et compléter le volume final du mélange avec de l'eau bi-distillée ;
   g) contrôler la teneur en oxygène de la solution et procéder à une désoxygénation au moyen de la vapeur blanche si la teneur en oxygène est supérieure à 0, 2 ppm ;
   h) faire passer la solution de paracétamol à travers un premier filtre (14) de 0,45 $\mu$m puis un deuxième filtre (14) de 0,22 $\mu$m ;
   i) procéder à l'étape de conditionnement consistant à remplir dans une poche souple (3) la solution de paracétamol filtrée à l'étape h), et à enfermer de manière étanche ladite poche (3) dans un suremballage (2) avec un absorbant d'oxygène (4) ; et

   **en ce que** les étapes a) à h) sont réalisées dans ledit réacteur étanche (15) sous atmosphère désoxygénée à la vapeur blanche d'eau bi-distillée.

**6.** Procédé selon la revendication 5, **caractérisé en ce que** la poche souple (3) contenant la solution de paracétamol comporte un espace de tête d'un volume d'air limité compris entre 5 et 10 cm$^3$.

**7.** Procédé selon la revendication 5 ou 6, **caractérisé en ce qu'**à l'étape i) on réalise un vide partiel dans ledit suremballage (2) avant d'enfermer ladite poche souple (3) et ledit absorbant (4) dans le suremballage (2).

**8.** Procédé selon l'une des revendications 5 à 7, **caractérisé en ce que** l'étape i) du procédé est réalisée dans une salle de conditionnement de classe particulaire A ou B de manière à éviter l'étape de stérilisation à l'autoclave.

**9.** Procédé selon l'une des revendications 5 à 7, **caractérisé en ce que** l'étape i) du procédé étant réalisée dans une salle de conditionnement de classe particulaire C, le procédé comprend une étape supplémentaire de stérilisation par autoclavage de la solution conditionnée après l'étape i).

**Claims**

**1.** A formulation for an injectable and ready-to-use paracetamol solution, **characterised in that** said paracetamol solution is only composed of paracetamol, bi-distilled water and a buffer agent, said formulation having a maximum paracetamol concentration of 1 g/100 ml, a pH of between 5.5 and 6.5 and an oxygen content of less than 0.2 ppm.

**2.** A formulation according to claim 1, **characterised in that** the buffer agent is a buffer agent couple composed of sodium acetate and acetic acid.

**3.** A formulation according to claim 1 or 2, **characterised in that** the solution has a pH of 6.

**4.** A formulation according to one of claims 1 to 3, **characterised in that** it contains 1 g/100 ml of paracetamol.

**5.** A method for preparing and packaging a paracetamol solution according to one of claims 1 to 4 by means of a hermetically sealed reactor (15), said reactor being composed of a vessel (7) hermetically connected to a bi-distilled water distribution circuit (8), an injection circuit for white vapour of bi-distilled water (9), a dissolution circuit (11) for paracetamol and sodium acetate and a solution transfer circuit (10), **characterised in that** said method comprises the following steps consisting in:

a) introducing a volume of bi-distilled water into the vessel (7) via the bi-distilled water distribution circuit (8), said vessel being previously cleaned with white vapour of bi-distilled water;
b) cooling the volume of water to a temperature of between 30°C and 35°C;
c) checking the content of oxygen dissolved in the volume of water and performing deoxygenation by means of white vapour of bi-distilled water if the oxygen content exceeds 0.2 ppm;
d) introducing the paracetamol and sodium acetate in powder form into said dissolution circuit (11) by aspiration, with the paracetamol and sodium acetate then being dissolved in the dissolution circuit by means of an ultra-dispersing device (16);
e) returning the mixture obtained at the dissolution circuit outlet into the vessel via the transfer circuit (10),
f) adjusting the pH of the solution with glacial acetic acid and complementing the final volume of the mixture with bi-distilled water;
g) checking the oxygen content of the solution and performing deoxygenation by means of the white vapour if the oxygen content exceeds 0.2 ppm;
h) transferring the paracetamol solution through a first 0.45 $\mu$m filter (14) then through a second 0.22 $\mu$m filter (14);
i) performing the packaging step consisting in filling a flexible pocket (3) with the paracetamol solution filtered in step h), and in hermetically sealing said pocket (3) in an over-packaging (2) with an oxygen absorbent (4); and

wherein steps a) to h) are performed in said hermetically sealed reactor (15) in an atmosphere deoxygenated using white vapour of bi-distilled water.

**6.** A method according to claim 5, **characterised in that** the flexible pocket (3) containing the paracetamol solution comprises a head space with a limited air volume of between 5 and 10 cm$^3$.

**7.** A method according to claim 5 or 6, **characterised in that** in step i), a partial vacuum is created in said over-packaging (2) before enclosing said flexible pocket (3) and said absorbent (4) in the over-packaging (2).

8. A method according to one of claims 5 to 7, **characterised in that** step i) of the method is performed in a packaging room with a cleanliness class A or B so as to avoid the autoclave sterilisation step.

9. A method according to one of claims 5 to 7, **characterised in that** where step i) of the method is performed in a packaging room with a cleanliness class C, the method comprises an additional step consisting in an autoclave sterilisation of the packaged solution after step i).

**Patentansprüche**

1. Formulierung einer gebrauchsfertigen injizierbaren Paracetamol-Lösung, **dadurch gekennzeichnet, dass** die besagte Paracetamol-Lösung ausschließlich aus Paracetamol, aus bidestilliertem Wasser und aus einem Puffersystem besteht, und die besagte Formulierung eine maximale Paracetamol-Konzentration von 1 g/100 ml, einen pH-Wert zwischen 5,5 und 6,5 und einen Sauerstoffgehalt von unter 0,2 ppm aufweist.

2. Formulierung nach Anspruch 1, **dadurch gekennzeichnet, dass** das Puffersystem ein Puffersystempaar bestehend aus Natriumacetat und Essigsäure ist.

3. Formulierung nach Anspruch 1 oder 2, **dadurch gekennzeichnet, dass** der pH-Wert der Lösung 6 ist.

4. Formulierung nach einem der Ansprüche 1 bis 3, **dadurch gekennzeichnet, dass** sie 1 g/100 ml an Paracetamol enthält.

5. Verfahren zur Herstellung und Konditionierung einer Paracetamol-Lösung nach einem der Ansprüche 1 bis 4 mithilfe eines dichten Reaktionsgefäßes (15), wobei das besagte Reaktionsgefäß aus einem Behälter (7) besteht, der in dichter Form mit einem Zuführungskreis für bidestilliertes Wasser (8), einem Injektionskreis für weißen Dampf aus bidestilliertem Wasser (9), einem Kreis zum Lösen (11) von Paracetamol und Natriumacetat und einem Kreis für den Transfer (10) der Lösung verbunden ist, **dadurch gekennzeichnet, dass** das besagte Verfahren die folgenden Schritte umfasst:

   a) das Einbringen eines Volumens an bidestilliertem Wasser über den Zuführungskreis für bidestilliertes Wasser (8) in den Behälter (7), wobei der besagte Behälter vorab mit weißem Dampf aus bidestilliertem Wasser gereinigt wird;
   b) das Kühlen des Wasservolumens auf eine Temperatur zwischen 30 °C und 35 °C;
   c) die Kontrolle des Gehalts an gelöstem Sauerstoff im Wasservolumen und die Vornahme einer Sauerstoffentziehung mithilfe des weißen Dampfes aus bidestilliertem Wasser, falls der Sauerstoffgehalt über 0,2 ppm liegt;
   d) das Einbringen durch Ansaugen von Paracetamol und Natriumacetat in Pulverform in den besagten Lösungskreis (11), wobei das Paracetamol und das Natriumacetat anschließend mit einer Ultra-Dispergiervorrichtung (16) im Lösungskreis gelöst werden;
   e) die Rückführung der erhaltenen Mischung über den Transferkreis (10) zum Auslauf des Lösungskreises im Behälter,
   f) die Anpassung des pH-Werts der Lösung mithilfe der eiskalten Essigsäure und die Ergänzung des endgültigen Volumens der Mischung mit dem bidestillierten Wasser;
   g) die Kontrolle des Sauerstoffgehalts der Lösung und die Vornahme einer Sauerstoffentziehung mithilfe des weißen Dampfes aus bidestilliertem Wasser, falls der Sauerstoffgehalt über 0,2 ppm liegt;
   h) die Durchleitung der Paracetamol-Lösung durch einen ersten Filter (14) mit 0,45 $\mu$m, gefolgt von einem zweiten Filter (14) mit 0,22 $\mu$m;
   i) die Vornahme des Konditionierungsschrittes bestehend aus dem Einfüllen der in Schritt h) gefilterten Paracetamol-Lösung in einen flexiblen Beutel und aus dem dichten Einschließen des besagten Beutels (3) in eine Umverpackung (2) mit einem Sauerstoff-Absorber (4); und

   dadurch, dass die Schritte a) bis h) in dem besagten dichten Reaktionsgefäß (15) unter einer mit weißem Dampf aus bidestilliertem Wasser desoxidierten Atmosphäre durchgeführt werden.

6. Verfahren nach Anspruch 5, **dadurch gekennzeichnet, dass** der flexible Beutel (3) mit der Paracetamol-Lösung einen Kopfraum mit einem begrenzten Luftvolumen zwischen 5 und 10 cm$^3$ umfasst.

7. Verfahren nach Anspruch 5 oder 6, **dadurch gekennzeichnet, dass** man im Schritt i) ein Teilvakuum in der Umverpackung (2) erzeugt, bevor der besagte flexible Beutel (3) und der besagte Absorber (4) in die Umverpackung (2) eingeschlossen werden.

8. Verfahren nach einem der Ansprüche 5 bis 7, **dadurch gekennzeichnet, dass** der Schritt i) des Verfahrens in einem Konditionierungsraum der Partikelklasse A oder B durchgeführt wird, sodass der Schritt der Sterilisierung im Autoklav vermieden wird.

9. Verfahren nach einem der Ansprüche 5 bis 7, **dadurch gekennzeichnet, dass** das Verfahren, da der Schritt i) des Verfahrens in einem Konditionierungsraum der Partikelklasse C durchgeführt wird, einen zusätzlichen Schritt zur Sterilisierung der konditionierten Lösung im Autoklav nach dem Schritt i) umfasst.

EP 2 087 909 B1

Fig. 1

**Fig. 2**

6  4

1

5

2

3

**Fig. 3**

4  201  301  3

2

O² O² O² O²

Solution de paracétamol 1%
dans eau bidistillée

Fe→ Fe²+ + 2e-
O²+ 2H²O + 4e-→4OH-
Fe²+ + 20H-→Fe(OH)²
4Fe(OH)²+ O²+ 2H²O→4Fe(OH)3

Perméabilité oxygène
suremballage:
1ml
/m²/24h/23°C/50%HR

Perméabilité oxygène
poche polypropylène:
950ml
/m²/24h/23°C/50%HR

## RÉFÉRENCES CITÉES DANS LA DESCRIPTION

**Documents brevets cités dans la description**

- WO 9805314 A